# EUROPEAN PATENT APPLICATION

(11) **EP 4 596 073 A1**
(43) Date of publication of application: **06.08.2025**
(21) Application number: 23872389.4
(22) Date of filing: 27.09.2023
(51) Int. Cl.: A63H 11/00, A63H 3/00, A63H 5/00

(54) **ROBOT**

(30) Priority: 29.09.2022 JP 2022156761
(71) Applicant: Nitto Denko Corporation, Ibaraki-shi, Osaka 567-8680 (JP)
(72) Inventor: FUKUSHIMA, Rihito, Ibaraki-shi, Osaka 567-8680 (JP); YAMAUCHI, Kengo, Ibaraki-shi, Osaka 567-8680 (JP); KIYOSHIMA, Keita, Ibaraki-shi, Osaka 567-8680 (JP); SHIMIZU, Yusuke, Ibaraki-shi, Osaka 567-8680 (JP)
(74) Representative: Epping - Hermann - Fischer
(86) International application number: PCT/JP2023/035070
(87) International publication number: WO 2024/071167

(57) **Abstract**

A robot that can naturally acquire highly reliable biological information is provided. The robot according to one aspect of the present invention is a robot configured to acquire, in contact with or in proximity to a user, biological information of the user, and including an exterior member, a detector configured to acquire the biological information, using an electromagnetic wave, and a controller configured to control an operation of at least one of the robot or the detector such that at least one of an orientation, position, or pose of the detector relative to the user changes based on the biological information acquired by the detector.

## Description

### TECHNICAL FIELD

The present disclosure relates to a robot.

### BACKGROUND

Conventionally, a robot including a sensor and configured to collect biological information of a user is known. The biological information collected by the robot is used for grasping a physical state or psychological state of a user or the like.

As the above-described robot, in order to continuously enjoy health support without making the user aware of the diagnosis, a robot configured to acquire biological information of a user from a contact position, in response to the user contacting the device, without requiring the user to contact the device is disclosed (see, for example, Patent Document 1).

### Related Art Document

### Patent Document

[Patent Document 1] Japanese Patent No. 6519560

### SUMMARY OF THE INVENTION

### Problem to be solved by the invention

However, the robot described in Patent Document 1 acquires biological information in response to the user contacting the robot itself, and thus there is scope for improvement in terms of restricting, when acquiring biological information, the user to acquire biological information naturally. Additionally, there is a case in which highly reliable biological information cannot be acquired due to the orientation, position, or pose of a biological information acquisition means relative to the user, or shielding, such as clothing or baggage.

It is an object of the present invention to provide a robot configured to naturally acquire highly reliable biological information.

### Means for Solving the Problem

The robot according to one aspect of the present invention is a robot configured to acquire, in contact with or in proximity to a user, biological information of the user, and includes an exterior member, a detector configured to acquire the biological information by using an electromagnetic wave, and a controller configured to control, based on the biological information acquired by the detector, an operation of at least one of the robot or the detector, such that at least one of an orientation, position, or pose of the detector relative to the user changes.

### Effect of the invention

According to the present invention, a robot that can naturally acquire highly reliable biological information can be provided.

### BRIEF DESCRIPTION OF THE DRAWINGS

[FIG. 1] FIG. 1 is a perspective view illustrating a robot according to an embodiment.
[FIG. 2] FIG. 2 is a side view of the robot of FIG. 1.
[FIG. 3] FIG. 3 is a cross-sectional view along the III-III cut line in FIG. 2.
[FIG. 4] FIG. 4 is a view illustrating a configuration of a camera according to the embodiment.
[FIG. 5] FIG. 5 is a view illustrating a configuration of a vital sensor according to the embodiment.
[FIG. 6] FIG. 6 is a view illustrating a hardware configuration of a controller according to the embodiment.
[FIG. 7] FIG. 7 is a block diagram illustrating a functional configuration of the controller according to the embodiment.
[FIG. 8] FIG. 8 is a flowchart illustrating the processing by the control unit according to an embodiment.
[FIG. 9] FIG. 9 is a diagram showing a first example of the holding state of a robot according to an embodiment.
[FIG. 10] FIG. 10 is a diagram showing a second example of the holding state of a robot according to an embodiment.

### DESCRIPTION OF THE EMBODIMENTS

In the following, embodiments of the present disclosure will be described in detail with reference to the drawings. In the drawings, the same components are denoted by the same reference symbols, and duplicated description will be appropriately omitted.

The embodiments described below are examples of a robot for embodying the technical idea of the present disclosure, and the present disclosure is not limited to the embodiments described below. The dimensions, materials, shapes, relative arrangements, and the like of the components described below are intended to illustrate the examples, not to limit the scope of the present disclosure thereto, unless otherwise specified. Additionally, the size, positional relationship, and the like of members illustrated in the drawings may be exaggerated for clarity of description.

### <Overall Configuration Example of Robot 100>

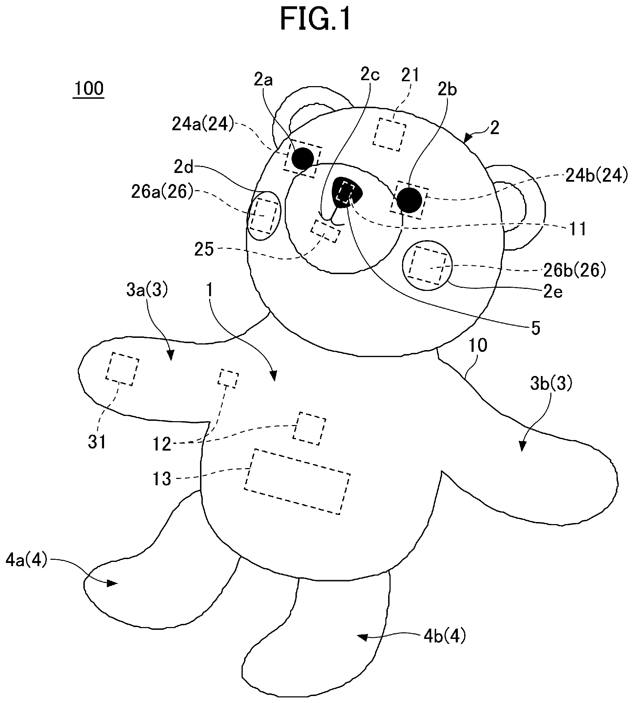

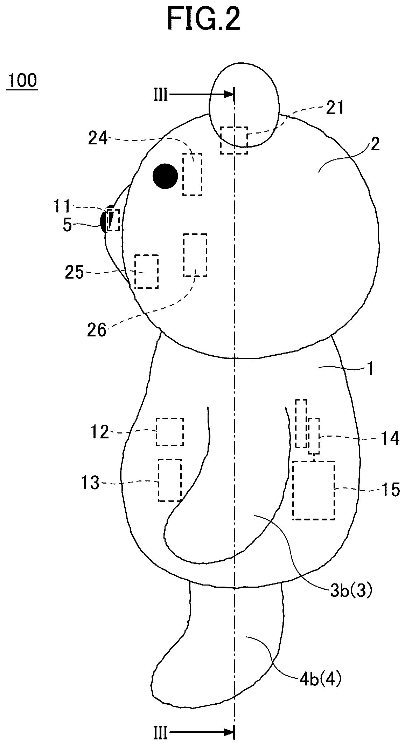

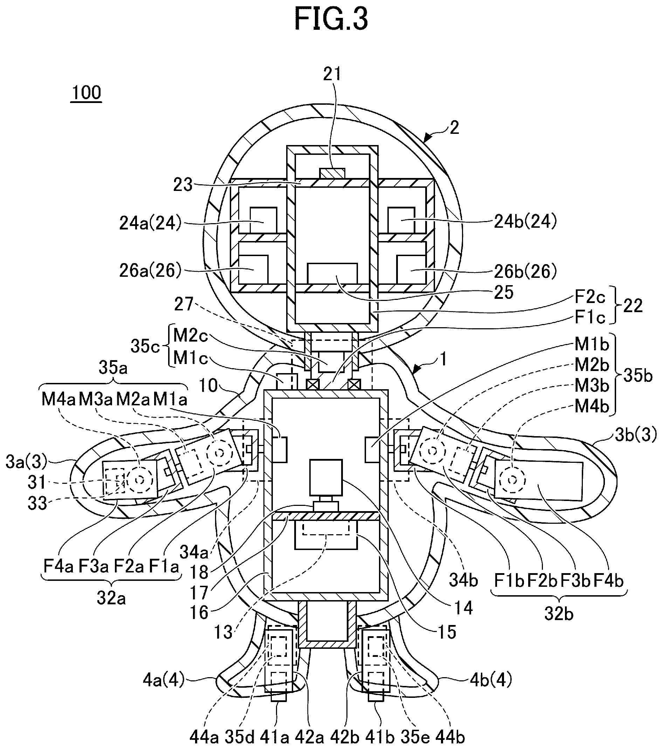

A configuration of a robot 100 according to an embodiment will be described with reference to FIGS. 1 to 3. FIG. 1 is a perspective view illustrating the robot 100 according to the embodiment. FIG. 2 is a side view of the robot 100. FIG. 3 is a cross-sectional view along a cutting line III-III in FIG. 2.

The robot 100 is a robot that includes exterior members 10 and that is configured to be driven by supplied power. The robot 100 described in the present specification as an example is a doll-type communication robot in the shape of a bear cub. The robot 100 is manufactured to have a size and weight suitable for being held by a user. Here, the user indicates a user of the robot 100. Typical examples of the user include a working adult living alone, a senior person whose child has moved out, and a frail elderly person who is a target of home healthcare. Here, the user may include a contact person who simply contacts the robot 100, such as an administrator of the robot 100, in addition to the user of the robot 100. Additionally, in the present specification, the term "hold" may be referred to as "carry".

In the present embodiment, the robot 100 can acquire the biological information of the user in contact with or in proximity to the user. The user corresponds to a "person" who is in contact with or in proximity to the robot 100. The robot 100 acquires the biological information of the user in a state where the vital sensor and the user are not in contact with each other, that is, in a non-contact state, by a vital sensor (a detector) configured to acquire the biological information using an electromagnetic wave. As a result, in comparison with a case where the robot 100 acquires the biological information in a state in which the vital sensor and the user are in contact with each other, the robot 100 can naturally acquire the biological information of the user by reducing a sense of restraint and resistance felt by the user.

Here, the vital sensor may be disposed inside the exterior member 10. This is because, in comparison with a case where the vital sensor is exposed from the surface of the robot 100, the vital sensor is not easily broken and the tactile feel of the robot 100 improves. With this, the user is more likely to secrete oxytocin by the interaction with the robot 100. The interaction between the robot 100 and the user indicates an action of the user and the robot 100 interacting with each other (an action of touching), such as rubbing, tapping, and hugging.

With respect to the above, when the distance between the vital sensor and the user is in proximity to each other and the biological information is acquired without contact, there are cases where the robot 100 cannot acquire the appropriate biological information if the orientation, position, and pose of the vital sensor relative to the user are not appropriate. In other words, the appropriate state is a state where shift of the orientation, position, and pose of the vital sensor relative to the user from a predetermined state is small. In other words, the appropriate biological information is highly reliable biological information. For example, in a case of acquiring biological information from the chest of the user and a case of acquiring biological information from the abdomen of the user, the dominance of the respiratory component changes, and thus the ratio of the respiration to the heartbeat changes, so that there are cases where highly reliable biological information cannot be acquired.

In the present embodiment, when highly reliable biological information cannot be acquired during a period of the interaction with the user, an operation of at least one of the robot 100 or the vital sensor is controlled so that the highly reliable biological information can be acquired. For example, the robot 100 performs an operation to prompt the user to modify a holding state of the robot 100, and continues to appeal by the operation until the holding state of the robot 100 by the user becomes a state in which the highly reliable biological information can be acquired. The robot 100 can naturally acquire the highly reliable biological information by the user modifying the holding state of the robot 100 according to the operation of the robot 100.

The exterior member 10 may have flexibility. The exterior member 10 contains, for example, a soft material that is comfortable to touch when the user of the robot 100 touches the robot 100. The exterior member 10 may have flexibility by containing at least one of an elastic material or a porous material. Specifically, as the material of the exterior member 10, a material containing an organic material, such as urethane foam, rubber, resin, or fiber can be used. The exterior member 10 preferably includes an exterior, such as a urethane foam material, having a heat insulating property, and a soft cloth material covering the outer surface of the exterior. The exterior member 10 has flexibility, for example, by containing at least one of an elastic material or a porous material, so that the user can feel the softness of the robot 100. With this, a restraining feeling and user resistance can be reduced, thereby promoting communication between the user and the robot 100.

The robot 100 includes, for example, a trunk 1, a head 2, arms 3, and legs 4. The head 2 has a right eye 2a, a left eye 2b, a mouth 2c, a right cheek 2d, and a left cheek 2e. The arms 3 include a right arm 3a and a left arm 3b, and the legs 4 include a right leg 4a and a left leg 4b. Here, the trunk 1 corresponds to a robot main body.

The head 2, the arm 3, and the leg 4 correspond to a drive section connected to the robot main body so as to be relatively displaceable. The drive section in the present embodiment includes the arm 3 connected to the robot main body in the robot 100 so as to be relatively displaceable.

In the present embodiment, the arm 3 is configured to be displaceable with respect to the trunk 1. For example, when the robot 100 is held by the user, the robot 100 displaces the right arm 3a and the left arm 3b to contact a neck, a trunk, or the like of the user so as to hold the user. This operation allows the user to feel an affinity for the robot 100, and thus promotes the interaction between the user and the robot 100.

The trunk 1, the head 2, the arms 3, and the legs 4 are all covered with the exterior members 10. The exterior member at the trunk 1 and the exterior member at the arm 3 are integrated, and the exterior members at the head 2 and the leg 4 are separated from the exterior member at the trunk 1 and the arm 3. However, the embodiments are not limited to these configurations, and for example, only a portion of the robot 100 that is likely to be contacted by the user may be covered by the exterior member 10. Additionally, at least one of the exterior members 10 at the trunk 1, the head 2, the arm 3, and the leg 4 may be separated from the other exterior members. Further, a portion in the head 2, the arm 3, and the leg 4 that does not displace may be configured only by the exterior member 10 without including a component such as a sensor therein.

The robot 100 includes a camera 11, a tactile sensor 12, a controller 13, a vital sensor 14, a battery 15, an adjustable mechanism 18, a first capacitive sensor 21, and a second capacitive sensor 31 inside the exterior members 10. Additionally, the robot 100 includes the tactile sensor 12, the controller 13, the vital sensor 14, the battery 15, and the adjustable mechanism 18 inside the exterior member 10 at the trunk 1. Additionally, the robot 100 includes the camera 11, the first capacitive sensor 21 inside the exterior member 10 at the head 2, and the second capacitive sensor 31 inside the exterior member 10 at the arm 3.

Additionally, the robot 100 includes displays 24, a speaker 25, and lights 26 inside the exterior member 10 at the head 2. Additionally, the robot 100 includes the displays 24 inside the exterior member 10 at the right eye 2a and the left eye 2b. Additionally, the robot 100 includes the speaker 25 inside the exterior member 10 at the mouth 2c, and the lights 26 inside the exterior member 10 at the right cheek 2d and the left cheek 2e.

More specifically, as illustrated in FIG. 3, the robot 100 includes a trunk frame 16 and a trunk mounting base 17 inside the exterior member 10 at the trunk 1. Additionally, the robot 100 includes a head frame 22 and a head mounting base 23 inside the exterior member 10 at the head 2. Further, the robot 100 includes a right arm frame 32a and a right arm mounting base 33 inside the exterior member 10 at the right arm 3a, and a left arm frame 32b inside the exterior member 10 at the left arm 3b. In addition, the robot 100 includes a right leg frame 42a inside the exterior member 10 at the right leg 4a, and a left leg frame 42b inside the exterior member 10 at the left leg 4b.

The trunk frame 16, the head frame 22, the right arm frame 32a, the left arm frame 32b, the right leg frame 42a, and the left leg frame 42b are structures each formed by combining multiple columnar members. The trunk mounting base 17, the head mounting base 23, and the right arm mounting base 33 are plate members having placement surfaces. The trunk mounting base 17 is fixed to the trunk frame 16, the head mounting base 23 is fixed to the head frame 22, and the right arm mounting base 33 is fixed to the right arm frame 32a. Here, the trunk frame 16, the head frame 22, the right arm frame 32a, the left arm frame 32b, the right leg frame 42a, and the left leg frame 42b may be formed in a box shape including multiple plate members.

The right arm frame 32a is connected to the trunk frame 16 via a right arm connection mechanism 34a, and is driven by a right arm servo motor 35a, thereby being relatively displaceable with respect to the trunk frame 16. The shift of the right arm frame 32a causes the right arm 3a to displace relative to the trunk 1. The right arm connection mechanism 34a preferably includes a reduction gear that increases the output torque of the right arm servo motor 35a, for example.

In the present embodiment, the right arm frame 32a is configured by a multi-joint robot arm including multiple frame members and multiple connection mechanisms. For example, the right arm frame 32a includes a right shoulder frame F1a, a right upper arm frame F2a, a right elbow frame F3a, and a right forearm frame F4a. The trunk frame 16, the right shoulder frame F1a, the right upper arm frame F2a, the right elbow frame F3a, and the right forearm frame F4a are connected to each other via respective connection mechanisms.

The right arm servo motor 35a is a generic term of multiple servo motors. For example, the right arm servo motor 35a includes a right shoulder servo motor M1a, a right upper arm servo motor M2a, a right elbow servo motor M3a, and a right forearm servo motor M4a. The right shoulder servo motor M1a rotates the right shoulder frame F1a about a rotation axis perpendicular to the trunk frame 16. The right upper arm servo motor M2a rotates the right upper arm frame F2a about a rotation axis perpendicular to a rotation axis of the right shoulder frame F1a. The right elbow servo motor M3a rotates the right elbow frame F3a about a rotation axis perpendicular to a rotation axis of the right upper arm frame F2a. The right forearm servo motor M4a rotates the right forearm frame F4a about a rotation axis perpendicular to a rotation axis of the right elbow frame F3a.

The left arm frame 32b is connected to the trunk frame 16 via a left arm connection mechanism 34b, and is driven by a left arm servo motor 35b, thereby being relatively displaceable with respect to the trunk frame 16. The shift of the left arm frame 32b causes the left arm 3b to displace relative to the trunk 1. The left arm connection mechanism 34b preferably includes a reduction gear that increases the output torque of the left arm servo motor 35b, for example.

In the present embodiment, the left arm frame 32b is configured by a multi-joint robot arm including multiple frame members and multiple connection mechanisms. For example, the left arm frame 32b includes a left shoulder frame F1b, a left upper arm frame F2b, a left elbow frame F3b, and a left forearm frame F4b. The trunk frame 16, the left shoulder frame F1b, the left upper arm frame F2b, the left elbow frame F3b, and the left forearm frame F4b are connected to each other via respective connection mechanisms.

The left arm servo motor 35b is a generic term of multiple servo motors. For example, the left arm servo motor 35b includes a left shoulder servo motor M1b, a left upper arm servo motor M2b, a left elbow servo motor M3b, and a left forearm servo motor M4b. The left shoulder servo motor M1b rotates the left shoulder frame F1b about a rotation axis perpendicular to the trunk frame 16. The left upper arm servo motor M2b rotates the left upper arm frame F2b about a rotation axis perpendicular to a rotation axis of the left shoulder frame F1b. The left elbow servo motor M3b rotates the left elbow frame F3b about a rotation axis perpendicular to a rotation axis of the left upper arm frame F2b. The left forearm servo motor M4b rotates the left forearm frame F4b about a rotation axis perpendicular to a rotation axis of the left elbow frame F3b.

As described above, the arm 3 includes the four-axis joints, thereby enabling the robot 100 to realize a realistic operation. The realistic operation refers to a natural motion as a motion of animals including humans. In the present embodiment, the realistic operation corresponds to a natural motion of the robot 100 as a bear cub.

The head frame 22 is connected to the trunk frame 16 via a head connection mechanism 27, and is driven by a head servo motor 35c, thereby being relatively displaceable with respect to the trunk frame 16. The shift of the head frame 22 causes the head 2 to displace relative to the trunk 1. The head connection mechanism 27 preferably includes a reduction gear that increases the output torque of the head servo motor 35c, for example.

In the present embodiment, the head frame 22 includes a neck frame F1c and a face frame F2c. The trunk frame 16, the neck frame F1c, and the face frame F2c are connected to each other via respective connection mechanisms.

The head servo motor 35c is a generic term of multiple servo motors. For example, the head servo motor 35c includes a neck servo motor M1c and a face servo motor M2c. The neck servo motor M1c rotates the neck frame F1c about a rotation axis perpendicular to the trunk frame 16. The face servo motor M2c rotates the face frame F2c around a rotation axis perpendicular to a rotation axis of the neck frame F1c. As described, the robot 100 can realize a more realistic operation because the head 2 includes the two-axis joints.

The right leg frame 42a is connected to the trunk frame 16 via a right leg connection mechanism 44a, and includes a right leg wheel 41a on the bottom side. In order to stabilize the posture of the robot 100, the robot 100 preferably includes two right leg wheels 41a in the front-rear direction of the right leg frame 42a. The right leg wheel 41a is driven by a right leg servo motor 35d, thereby being rotatable about a rotation axis perpendicular to the front-rear direction of the right leg frame 42a. The rotation of the right leg wheel 41a enables the robot 100 to travel. The right leg connection mechanism 44a preferably includes a reduction gear that increases the output torque of the right leg servo motor 35d, for example.

The left leg frame 42b is connected to the trunk frame 16 via a left leg connection mechanism 44b, and includes a left leg wheel 41b on the bottom side. In order to stabilize the posture of the robot 100, the robot 100 preferably includes two left leg wheels 41b in the front-rear direction of the left leg frame 42b. The left leg wheel 41b is driven by the left leg servo motor 35e, thereby being rotatable about a rotation axis perpendicular to the front-rear direction of the left leg frame 42b. The rotation of the left leg wheel 41b enables the robot 100 to travel. The left leg connection mechanism 44b preferably includes a reduction gear that increases the output torque of a left leg servo motor 35e, for example.

In the present embodiment, the robot 100 moves forward or backward by simultaneously rotating the right leg wheel 41a and the left leg wheel 41b forward or backward. The robot 100 turns right or left by braking one of the right leg wheel 41a or the left leg wheel 41b with a brake and turning the other forward or backward. As described above, the robot 100 can realize a more realistic operation by the leg 4.

The tactile sensor 12, the controller 13, the vital sensor 14, and the battery 15 are fixed to the trunk mounting base 17. The controller 13 and the battery 15 are fixed to the side of the trunk mounting base 17 opposite to the side to which the tactile sensor 12 and the vital sensor 14 are fixed. Here, the arrangement of the controller 13 and the battery 15 is due to the space available on the trunk mounting base 17 and is not limited to the above described arrangement. However, if the battery 15 is fixed to the side of the trunk mounting base 17 opposite to the side to which the tactile sensor 12 and the vital sensor 14 are fixed, the center of gravity of the robot 100 is lowered because the battery 15 is heavier than other components. The center of gravity of the robot 100 is preferably low because at least one of the position or the posture of the robot 100 is stabilized and at least one of charging or replacement of the battery 15 is easily performed.

The first capacitive sensor 21 is fixed to the head mounting base 23, and the second capacitive sensor 31 is fixed to the right arm mounting base 33. The display 24 includes a right-eye display 24a and a left-eye display 24b. The right-eye display 24a, the left-eye display 24b, and the speaker 25 are fixed to the head frame 22. The lights 26 include a right-cheek light 26a and a left-cheek light 26b. The right-cheek light 26a and the left-cheek light 26b are fixed to the head frame 22.

Here, the tactile sensor 12, the controller 13, the vital sensor 14, the battery 15, the first capacitive sensor 21, the second capacitive sensor 31, and the like can be fixed by a screw member, an adhesive member, or the like. Additionally, the right-eye display 24a, the left-eye display 24b, the speaker 25, the right-cheek light 26a, the left-cheek light 26b, and the like can also be fixed by screw members, adhesive members, or the like.

The materials of the trunk frame 16, the trunk mounting base 17, the head frame 22, the head mounting base 23, the right arm frame 32a, the right arm mounting base 33, and the left arm frame 32b are not particularly limited, and a resin material, a metallic material, or the like can be used. However, from the viewpoint of ensuring strength during driving, it is preferable to use a metallic material, such as aluminum, for the trunk frame 16, the right arm frame 32a, and the left arm frame 32b. If the strength can be obtained, it is preferable to use a resin material for the material of each of these parts in order to reduce the weight of the robot 100. The materials of the trunk mounting base 17, the head frame 22, the head mounting base 23, the right arm mounting base 33, and the left arm frame 32b are not particularly limited, and a resin material or a metallic material can be used. However, from the viewpoint of reducing the weight of the robot 100, it is preferable to use a resin material.

The controller 13 controls the operation of the entirety of the robot 100. In the present embodiment, in particular, the controller 13 controls the operation of at least one of the robot 100 or the vital sensor 14 such that at least one of the orientation, position, or pose of the vital sensor 14 relative to the user changes based on the biological information acquired by the vital sensor 14. For example, the controller 13 can control the operation of the robot 100 so as to provide a notification to the user that at least one of the orientation, position, or pose of the vital sensor 14 relative to the user is shifted from a predetermined state. The notification by the operation of the robot 100 to the user includes not only an operation that explicitly provides a notification with a message or the like using the display 24, the speaker 25, or the like, but also an operation that implicitly provides a notification with a message or the like by flapping the arm 3, the leg 4, or the like, or tapping the user by the arm 3.

The controller 13 is communicably connected to each of the camera 11, the tactile sensor 12, the vital sensor 14, the first capacitive sensor 21, the second capacitive sensor 31, the right arm servo motor 35a, and the left arm servo motor 35b by wire or wirelessly. Additionally, the controller 13 is communicably connected to each of the head servo motor 35c, the right leg servo motor 35d, and the left leg servo motor 35e by wire or wirelessly. Further, the controller 13 is communicably connected to each of the right-eye display 24a, the left-eye display 24b, the speaker 25, the right-cheek light 26a, and the left-cheek light 26b by wire or wirelessly.

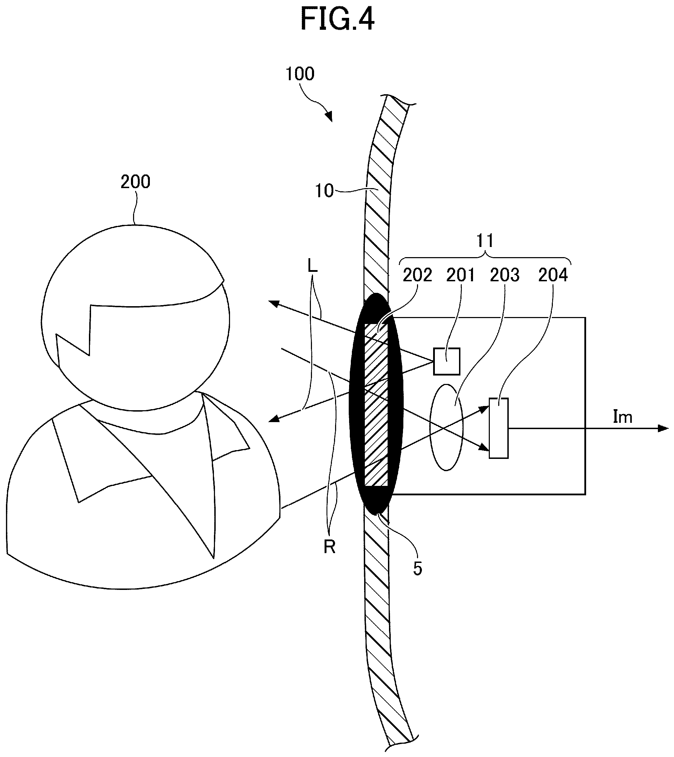

The camera 11 is an image sensor configured to output a captured image of the surroundings of the robot 100 to the controller 13. The camera 11 is an example of a capturing section configured to capture an image of the user. The camera 11 is disposed at a position corresponding to a nose 5 of the bear cub inside the exterior member 10. The camera 11 can be fixed by an adhesive member or the like. Here, a configuration of the camera 11 will be described in detail with reference to FIG. 4, separately.

The tactile sensor 12 is a sensor element configured to acquire information felt by a tactile sense inherent in a human hand or the like, convert the information into a tactile signal, which is an electrical signal, and output the tactile signal to the controller 13. For example, the tactile sensor 12 converts information on pressure or vibration generated by the user contacting the robot 100 into a tactile signal by a piezoelectric element, and outputs the tactile signal to the controller 13. The tactile signal output from the tactile sensor 12 is used to detect whether a user has come into contact with or come in proximity to the robot 100.

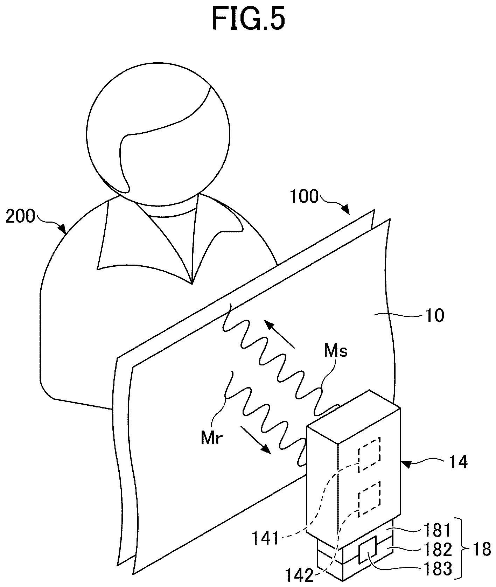

The vital sensor 14 is an example of the detector configured to acquire the biological information of the user, using an electromagnetic wave. The vital sensor 14 is supported by the adjustable mechanism 18. The adjustable mechanism 18 is fixed on the trunk mounting base 17 by a screw member, an adhesive member, or the like. Here, the configuration of the vital sensor 14 and the adjustable mechanism 18 will be described in detail separately with reference to FIG. 5.

The first capacitive sensor 21 and the second capacitive sensor 31 are sensor elements configured to output, to the controller 13, a capacitance signal obtained by detecting, based on a change in capacitance, that the user has come into contact with or come in proximity to the robot 100. The first capacitive sensor 21 is preferably a rigid sensor having no flexibility in terms of stabilization of the exterior member 10. The arm 3 is a part that is easily touched by the user, and thus the second capacitive sensor 31 is preferably a sensor having flexibility including a conductive thread or the like from the viewpoint of improving the touch feeling. The capacitance signals output from the first capacitive sensor 21 and the second capacitive sensor 31 are used to detect that the user has contacted or approached the robot 100.

The right-eye display 24a and the left-eye display 24b are display modules configured to display character strings, such as characters, numerals, and symbols, or images in response to a command from the controller 13. The right-eye display 24a and the left-eye display 24b are examples of displays provided in the eyes of the robot 100. The right eye 2a and the left eye 2b correspond to the eyes of the robot 100. The right-eye display 24a and the left-eye display 24b are configured by, for example, liquid crystal display modules. The character strings or images displayed on the right-eye display 24a and the left-eye display 24b may be used to express the emotion of the robot 100 or the like.

The speaker 25 is a speaker unit configured to amplify an audio signal from the controller 13 and emit sound. The speaker 25 is an example of a sound generator provided in the robot 100. The sound emitted from the speaker 25 is a word (language), or a call and vocalization, which are not words, of the robot 100, and may be used to express the emotion of the robot 100 or the like.

The right-cheek light 26a and the left-cheek light 26b are examples of a light emitter provided on the head 2 of the robot 100. The right-cheek light 26a and the left-cheek light 26b are light modules configured to change at least one of a blinking speed, luminance, or a light color. The right-cheek light 26a and the left-cheek light 26b are configured of, for example, light emitting diode (LED) light modules.

The battery 15 is a power supply configured to supply power to each of the camera 11, the tactile sensor 12, the controller 13, the vital sensor 14, the first capacitive sensor 21, the second capacitive sensor 31, the right arm servo motor 35a, and the left arm servo motor 35b. Additionally, the battery 15 supplies power to each of the head servo motor 35c, the right leg servo motor 35d, and the left leg servo motor 35e. Further, the battery 15 supplies power to each of the right-eye display 24a, the left-eye display 24b, the speaker 25, the right-cheek light 26a, and the left-cheek light 26b. Various secondary batteries, such as a lithium ion battery and a lithium polymer battery can be used as the battery 15.

Here, installation positions of various sensors, such as the tactile sensor 12, the first capacitive sensor 21, and the second capacitive sensor 31 in the robot 100 can be appropriately changed. Additionally, various sensors, such as the tactile sensor 12, the first capacitive sensor 21, and the second capacitive sensor 31 may be disposed outside the robot 100 and may transmit necessary information to the robot 100 or an external device wirelessly.

Additionally, the robot 100 does not necessarily include the controller 13 inside the exterior member 10, and the controller 13 can communicate with each device from the outside of the exterior member 10 via wireless connection. The battery 15 can supply power to each component from the outside of the exterior member 10.

In the present embodiment, a configuration in which the head 2, the arm 3, and the leg 4 are displaceable is described as an example, but the present embodiment is not limited thereto, and at least one of the head 2, the arm 3, or the leg 4 may be displaceable. Additionally, the arm 3 is configured by a four-axis multi-joint robot arm, but may be configured by a six-axis multi-joint robot arm. Further, the arm 3 is preferably connectable to an end effector, such as a hand. Additionally, the leg 4 is configured by a wheel system, but can be configured by a crawler system, a leg system, or the like.

The configuration and shape of the robot 100 are not limited to those examples described in the present embodiment, and can be appropriately changed according to the preference of the user, the use form of the robot 100, and the like. For example, the robot 100 may be, instead of a form of imitating a bear cub, in a form of another living being, a form of a human, such as a humanoid, or the like. Additionally, the robot 100 may be in a form of a mobile device, such as a drone or a vehicle, including at least one of an arm, a display, a speaker, a light, or the like.

<Example of Configuration of Camera 11>

FIG. 4 is a view illustrating an example of the configuration of the camera 11. The camera 11 includes an imaging light source 201, a wavelength filter 202, a lens 203, and an imaging element 204. The camera 11 is disposed near a surface of the robot 100 while being camouflaged so as not to be visually recognized from the outside of the robot 100.

The imaging light source 201 irradiates a user 200 with irradiation light L having a predetermined peak wavelength. Although the predetermined peak wavelength is not particularly limited, the predetermined peak wavelength is preferably non-visible light, such as near-infrared light from the perspective of making the irradiation light less visible. The wavelength filter 202 is an optical element configured to transmit light having a wavelength near the peak wavelength in the irradiation light L from the imaging light source 201. The lens 203 forms an image of the user 200 or the like on an imaging surface of the imaging element 204 by using reflected light R, by the user 200 or the like, of the irradiation light L from the imaging light source 201. The imaging element 204 outputs, to the controller 13, a captured image Im obtained by capturing the image formed by the lens 203. The imaging element may be a charge coupled device (CCD), a complementary metal-oxide semiconductor (CMOS), or the like. The captured image may be either a still image or a moving image.

In the camera 11, the wavelength filter 202 is disposed to camouflage the lens 203 and the imaging element 204 so that they are not easily visible from the outside of the robot 100. The camera 11 captures an image of the user 200, using the light reflected by the user 200 and transmitted through the wavelength filter 202 among the light from the imaging light source 201. Additionally, the nose 5 is a portion that the user 200 has fewer opportunities to touch than the trunk 1, the head 2, the arm 3, or the like. Thus, by disposing the camera 11 in the nose 5, the user 200 is less likely to contact the camera 11. Therefore, an uncomfortable feeling, caused by the surface of the camera 11 being harder than the surface of the exterior member 10 when the user 200 contacts the robot 100, can be reduced. Even when the user 200 contacts the nose 5, if the tactile feeling of the nose 5 is different from that of the trunk 1, the head 2, the arm 3, or the like, it is not so unnatural, and an uncomfortable feeling of the touch can be reduced.

The position where the camera 11 is disposed is not limited to the nose 5, but may be another portion, such as the mouth, the eye, or the like, as long as the above-described same effect as that of the arrangement on the nose 5 is obtained. Additionally, the camera 11 is not limited to be disposed inside the exterior member 10, but may be disposed outside the exterior member 10. When the camera 11 is disposed outside the exterior member 10, it is not necessarily required to camouflage the camera 11 to be less visible, and therefore the camera 11 does not need to include the wavelength filter 202 for camouflage.

The camera 11 may be used as the detector configured to acquire the biological information, using electromagnetic waves. In this case, the irradiation light L and the reflected light R correspond to the electromagnetic waves used to acquire the biological information. In this case, the orientation, position, and pose of the camera 11 relative to the user 200 correspond to "orientation, position, and pose of the detector relative to the user".

The camera 11 may include a time of flight (TOF) camera configured to output a distance image around the robot 100 to the controller 13. Therefore, the captured image Im output from the camera 11 may include a three-dimensional captured image (a distance image) in addition to or instead of a two-dimensional captured image. The captured image Im may be used to detect the presence or approach of the user 200, detect the distance from the robot 100 to the user 200, authenticate the user 200, estimate the emotion or action of the user 200, or the like. The robot 100 may include multiple cameras 11 at multiple parts of the robot 100 for respective applications. In addition to the camera 11, the robot 100 may include a human sensor, such as an ultrasonic sensor, an infrared sensor, a millimeter wave radar, or light detection and raging (LiDAR) .

### <Configuration Example of Vital Sensor 14>

FIG. 5 is a diagram illustrating a configuration of the vital sensor 14. The vital sensor 14 is a microwave Doppler sensor including a microwave transmitter 141 and a microwave receiver 142. The microwave is an example of the electromagnetic wave.

The vital sensor 14 transmits a transmitted wave Ms, which is a microwave, toward the user 200 from the inside of the exterior member 10 by the microwave transmitter 141. The vital sensor 14 receives, by the microwave receiver 142, a reflected wave Mr of the transmitted wave Ms that is reflected by the user 200.

The vital sensor 14 detects, in a non-contact manner, a minute shift generated on a body surface due to the beating of the heart of the user 200 or the like, based on a difference between the frequency of the transmitted wave Ms and the frequency of the reflected wave Mr, by using the Doppler effect. The vital sensor 14 can acquire information, such as a heartbeat, respiration, a pulse wave, a blood pressure, or a pulse pressure, as biological information of the user 200 based on the detected minute shift, and output it to the controller 13. The biological information includes at least one of the pulse, the blood pressure, or respiration. The information on the breathing includes the respiratory count, rhythm, respiratory depth, and the like. The pulse wave includes a pulse, a pulse interval R-R, a pulse wave waveform, a pulse wave propagation speed, and the like.

The vital sensor 14 is not limited to the microwave Doppler sensor, and may be a sensor configured to detect a minute shift generated on a body surface by using a change in coupling between a human body and an antenna, or may be a sensor configured to use an electromagnetic wave other than the microwave, such as near-infrared light. Additionally, the vital sensor 14 may be a millimeter wave radar, a microwave radar, or the like. Further, the vital sensor 14 preferably includes a non-contact thermometer configured to detect infrared rays or the like transmitted from the user 200 in addition to the Doppler sensor. In this case, the vital sensor 14 detects biological information of the user 200 including information on at least one of a heartbeat (a pulse), respiration, a blood pressure, or a body temperature. Additionally, the vital sensor 14 may include multiple vital sensors respectively configured to detect multiple types of biological information, such as the heartbeat, the respiration, the pulse wave, the blood pressure, and the pulse pressure, and detect multiple types of biological information.

The vital sensor 14 is provided inside the exterior member 10, and thus the user 200 cannot visually recognize the vital sensor 14. This reduces resistance of the user 200 to the detection of the biological information, and enables the biological information to be smoothly acquired. Additionally, the vital sensor 14 can acquire the biological information in a non-contact manner, and thus can acquire the biological information even when the user 200 moves to some extent, unlike a contact sensor that requires the user 200 to be in contact with the same place for a certain period of time.

Additionally, by promoting the interaction between the user 200 and the robot 100 by the hug operation of the robot 100 or the like, the robot 100 is held by the user 200 and can acquire the biological information in a state of being in contact with or in proximity to the user 200. With this, the robot 100 can acquire highly reliable biological information in which noise is reduced.

The adjustable mechanism 18 supports the vital sensor 14 such that the orientation, position, and pose of the vital sensor 14 can be adjusted. The adjustable mechanism 18 includes a linear motion mechanism 181, a rotation mechanism 182, and a mechanism driving section 183. The adjustable mechanism 18 mounts the vital sensor 14 on a mounting surface.

The linear motion mechanism 181 is a mechanism part for adjusting the position of the vital sensor 14 in the three axial directions by moving the vital sensor 14 mounted on the mounting surface in a straight direction among the three axial directions orthogonal to each other. For the linear motion mechanism 181, a linear motion stage or the like in each of the three axial directions can be used. The rotation mechanism 182 is a mechanism part for adjusting the orientation and pose of the vital sensor 14 by rotating the vital sensor 14 mounted on the mounting surface in the three axial directions orthogonal to each other. For the rotation mechanism 182, a rotation stage or the like around each of the three axes can be used. The mechanism driving section 183 drives each of the linear motion mechanism 181 and the rotation mechanism 182 in response to a mechanism control signal from the controller 13. For the mechanism driving section 183, a motor or the like can be used. Here, the adjustable mechanism 18 does not necessarily have to move the vital sensor 14 in all three axial directions in a linear manner, nor rotate the vital sensor 14 in all three axial directions. The adjustable mechanism 18 may only be able to adjust at least one of the orientation, position, or pose of the vital sensor 14.

### <Configuration Example of Controller 13>

### (Hardware Configuration Example)

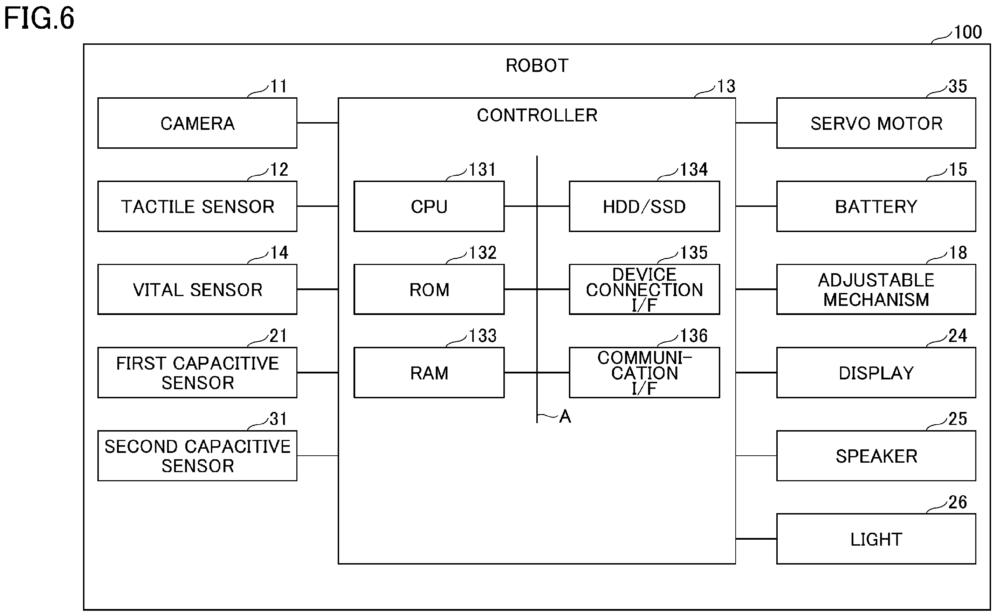

FIG. 6 is a block diagram illustrating an example of a hardware configuration of the controller 13. The controller 13 is constructed by a computer, and includes a central processing unit (CPU) 131, a read only memory (ROM) 132, and a random access memory (RAM) 133. Additionally, the controller 13 includes a hard disk drive/solid state drive (HDD/SSD) 134, a device connection interface (I/F) 135, and a communication I/F 136. These are communicably connected to each other via a system bus A.

The CPU 131 executes control processing including various arithmetic processing. The ROM 132 stores a program used to drive the CPU 131, such as an initial program loader (IPL). The RAM 133 is used as a work area of the CPU 131. The HDD/SSD 134 stores various information, such as programs, captured images acquired by the camera 11, detection information by various sensors, such as the biological information acquired by the vital sensor 14, the tactile signal acquired by the tactile sensor 12, and the like.

The device connection I/F 135 is an interface for connecting the controller 13 to various external devices. The external devices herein are the camera 11, the tactile sensor 12, the vital sensor 14, the first capacitive sensor 21, the second capacitive sensor 31, a servo motor 35, the battery 15, the adjustable mechanism 18, the light 26, and the like. Additionally, the external devices include the display 24, the speaker 25, and the like as illustrated in FIG. 1.

Here, the servo motor 35 is a generic term of the right arm servo motor 35a, the left arm servo motor 35b, the head servo motor 35c, the right leg servo motor 35d, and the left leg servo motor 35e. Additionally, the display 24 is a generic term for the right-eye display 24a and the left-eye display 24b. Further, the light 26 is a generic term for the right-cheek light 26a and the left-cheek light 26b.

The communication I/F 136 is an interface for communicating with an external device via a communication network or the like. For example, the controller 13 is connected to the Internet via the communication I/F 136 and communicates with the external device via the Internet.

Here, at least some of the functions realized by the CPU 131 may be realized by an electric circuit or an electronic circuit.

### (Functional Configuration Example)

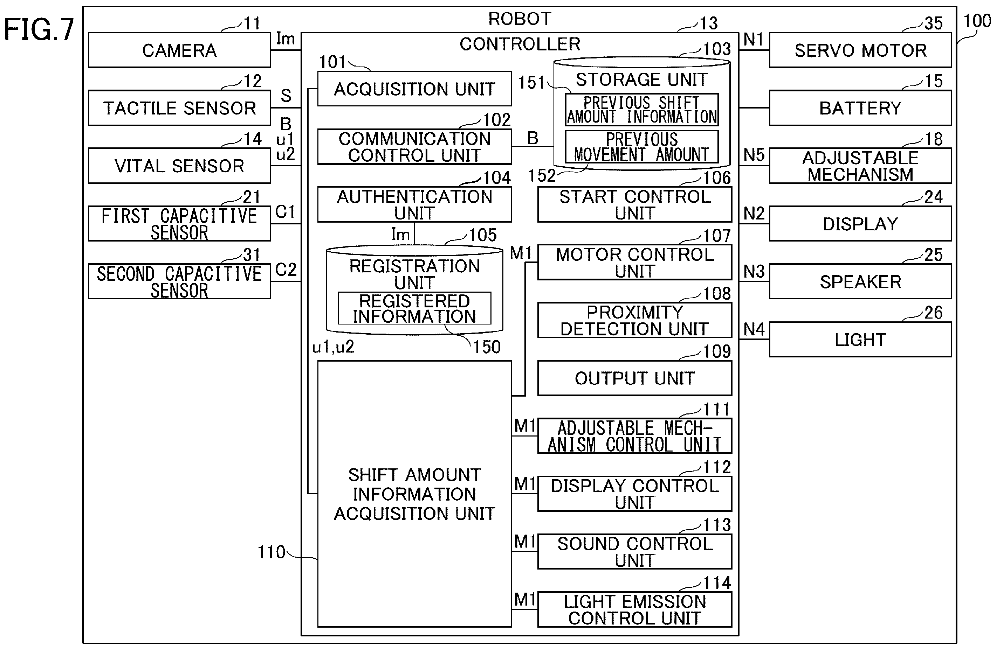

FIG. 7 is a block diagram illustrating an example of a functional configuration of the controller 13. The controller 13 includes an acquisition unit 101, a communication control unit 102, a storage unit 103, an authentication unit 104, a registration unit 105, a start control unit 106, a motor control unit 107, a proximity detector 108, and an output unit 109. Further, the controller 13 includes a shift amount information acquisition unit 110, an adjustable mechanism control unit 111, a display control unit 112, a sound control unit 113, and a light emission control unit 114. Here, the controller 13 may further include a function unit other than the above-described units.

The controller 13 can realize the functions of the acquisition unit 101 and the output unit 109 by the device connection I/F 135 or the like, and can realize the function of the communication control unit 102 by the communication I/F 136 or the like. Additionally, the controller 13 can realize the functions of the storage unit 103 and the registration unit 105 by a non-volatile memory, such as the HDD/SSD 134. Further, the controller 13 can realize the functions of the authentication unit 104, the start control unit 106, the motor control unit 107, and the proximity detector 108 by a processor, such as the CPU 131, executing processing defined in a program stored in a non-volatile memory, such as the ROM 132, or the like.

Additionally, the controller 13 can realize the functions of the shift amount information acquisition unit 110, the adjustable mechanism control unit 111, the display control unit 112, the sound control unit 113, and the light emission control unit 114 by a processor, such as the CPU 131, executing processing defined in a program stored in a non-volatile memory, such as the ROM 132, or the like. Here, some of the above-described functions of the controller 13 may be realized by an external device, such as a PC or a server, or may be realized by distributed processing between the controller 13 and the external device.

The acquisition unit 101 controls communication between the controller 13 and the camera 11 to acquire the captured image Im of the user 200 from the camera 11. Additionally, the acquisition unit 101 controls communication between the controller 13 and the tactile sensor 12 to acquire a tactile signal S from the tactile sensor 12. Further, the acquisition unit 101 controls communication between the controller 13 and the vital sensor 14 to acquire biological information B of the user 200 from the vital sensor 14.

Additionally, the acquisition unit 101 controls communication between the controller 13 and the first capacitive sensor 21 to acquire a first capacitance signal C1 from the first capacitive sensor 21. Additionally, the acquisition unit 101 controls communication between the controller 13 and the second capacitive sensor 31 to acquire a second capacitance signal C2 from the second capacitive sensor 31.

The communication control unit 102 controls communication with an external device via a communication network or the like. For example, the communication control unit 102 can transmit the captured image Im acquired by the camera 11, the biological information B acquired by the vital sensor 14, the tactile signal S acquired by the tactile sensor 12, and the like to the external device via the communication network.

The storage unit 103 stores highly reliable information among the biological information B acquired by the vital sensor 14. The storage unit 103 continuously stores the acquired biological information B while the acquisition unit 101 is acquiring the highly reliable biological information B from the vital sensor 14. Additionally, the storage unit 103 can store information obtained from the captured image Im by the camera 11, the tactile signal S from the tactile sensor 12, the first capacitance signal C1 from the first capacitive sensor 21, and the second capacitance signal C2 from the second capacitive sensor 31. Additionally, the storage unit 103 may store previous shift amount information 151, previous movement amount information 152, information on a first threshold, information on a second threshold, and the like, which will be described later.

The authentication unit 104 performs personal authentication of the user 200 based on the captured image Im of the user 200 captured by the camera 11. For example, the authentication unit 104 performs face authentication by referring to registered information 150 of a face image registered in advance in the registration unit 105, based on the captured image Im including the face of the user 200 captured by the camera 11. With this, the user 200 currently in contact with or in proximity to the robot 100 can be associated with the personal information registered in advance, and the biological information B acquired by the vital sensor 14 can be associated with the personal information. Additionally, the controller 13 can also perform control so as to stop the start of the acquisition of the biological information by the vital sensor 14 when the face image included in the captured image Im is not registered in the registration unit 105.

The start control unit 106 causes the vital sensor 14 to start acquiring the biological information B. For example, when the contact or proximity of the user 200 with respect to the robot 100 is detected by the proximity detector 108, the start control unit 106 turns on a switch or the like for supplying power from the battery 15 to the vital sensor 14. With this, the start control unit 106 causes the vital sensor 14 to start acquiring the biological information B.

The proximity detector 108 detects the contact or proximity of the user 200 with respect to the robot 100 based on the captured image Im and the like obtained by the camera 11. The proximity detector 108 may detect the distance from the robot 100 to the user 200 based on the captured image Im obtained by the camera 11. Additionally, the proximity detector 108 may detect the contact or proximity of the user 200 with respect to the robot 100 based on the first capacitance signal C1 or the second capacitance signal C2. Further, the proximity detector 108 may detect the contact or proximity of the user 200 with respect to the robot 100 based on the tactile signal S from the tactile sensor 12.

The shift amount information acquisition unit 110 acquires shift amount information M1, which is information on the shift amount of at least one of the orientation, position, or pose of the vital sensor 14 relative to the user 200 from a predetermined state. The shift amount information M1 includes information indicating the shift amount from the predetermined state, information related to the shift amount from the predetermined state, and the like.

For example, the vital sensor 14 outputs a first signal u1 related to the heart rate of the user 200 and a second signal u2 related to the respiration of the user 200 to the controller 13. The shift amount information acquisition unit 110 acquires information on a first shift amount related to the heart rate by comparing the predetermined first threshold related to the heart rate with the signal intensity of the first signal u1. Additionally, the shift amount information acquisition unit 110 acquires information on a second shift amount related to the respiration by comparing the predetermined second threshold related to the respiration with the signal intensity of the second signal u2. Here, the biological information B may be information including the first signal u1 and the second signal u2, or may be information itself indicated by the first signal u1 and the second signal u2. The biological information B may be information acquired by processing at least one of the first signal u1 or the second signal u2.

When none of the orientation, position, and pose of the vital sensor 14 is shifted from the predetermined state, the shift amount information acquisition unit 110 outputs the biological information B to an external device via the output unit 109. The external device herein is a PC, a display device, a storage device, an external server, or the like provided outside the controller 13. Additionally, the shift amount information acquisition unit 110 may output the biological information B to the storage unit 103 and store it. With respect to the above, when at least one of the orientation, position, or pose of the vital sensor 14 relative to the user 200 is shifted from the predetermined state, the shift amount information acquisition unit 110 outputs, to the motor control unit 107, the shift amount information M1 including the information on the first shift amount and the information on the second shift amount.

The above-described predetermined state indicates a state in which the orientation, position, and pose of the vital sensor 14 relative to the user 200 are appropriate so that the vital sensor 14 can acquire highly reliable biological information. For example, the predetermined state indicates a state in which the first signal u1 is less than or equal to the first threshold and the second signal u2 is less than or equal to the second threshold.

Here, the robot 100 may use the camera 11 as the detector, and the controller 13 may acquire the biological information B based on the image Im captured by the camera 11. For example, the controller 13 can acquire the biological information B by remote photoplethysmography (rPPG) based on the captured image Im. The rPPG is a technology to estimate the heart rate and respiration by analyzing a change in a skin color caused by blood flow. When the camera 11 is used as the detector, the shift amount information acquisition unit 110 acquires the shift amount information M1 based on the image Im captured by the camera 11. However, in order to simplify the description, the case where the vital sensor 14 is used as the detector will be described below as an example.

In response to the shift amount information M1 from the shift amount information acquisition unit 110, the motor control unit 107 controls the operation of the servo motor 35 so as to provide, to the user 200, a notification that at least one of the orientation, position, or pose of the vital sensor 14 relative to the user 200 is shifted from a predetermined state. The motor control unit 107 can control the operation of the arm 3 illustrated in FIG. 1 and the like by controlling the operation of the servo motor 35.

For example, when there is a shift of the vital sensor 14 relative to the user 200, the motor control unit 107 outputs, to the servo motor 35 via the output unit 109, a command N1 for "flapping" the arm 3. The motor control unit 107 can notify the user 200 of the shift by "flapping" the arm 3 through the servo motor 35. After starting the flapping operation of the arm 3, the motor control unit 107 stops the flapping operation after a predetermined time elapses. The user 200 recognizes that the holding state of the robot 100 is not appropriate based on the flapping of the arm 3, and corrects at least one of the orientation, position, or pose of the robot 100. The orientation, position, and pose of the vital sensor 14 relative to the user 200 are corrected according to the correction of the orientation, position, and pose of the robot 100.

The motor control unit 107 may control the operation of the arm 3 such that the movement amount of the arm 3 associated with flapping decreases as the shift of the vital sensor 14 relative to the user 200 decreases. This is because the user 200 can be made to recognize that the above-mentioned shift is reduced and that the correction of the orientation, position, and pose of the robot 100 by the user 200 is correct. For example, when the sum of the first shift amount and the second shift amount is less than the sum of the previous first shift amount and the previous second shift amount indicated by the previous shift amount information 151 stored in the storage unit 103, the motor control unit 107 sets the movement amount of the arm 3 for flapping less than the previous movement amount indicated by the previous movement amount information 152 stored in the storage unit 103. The reduction amount of the movement amount may be predetermined and stored in the storage unit 103 or the like. Here, the motor control unit 107 may shorten the flapping time as the shift of the vital sensor 14 relative to the user 200 decreases. Additionally, although the sum of the first shift amount and the second shift amount is exemplified as the shift amount in the present specification, the shift amount is not limited thereto. The larger amount of the first shift amount and the second shift amount may be used as the shift amount, or the average value of the first shift amount and the second shift amount may be used as the shift amount.

With respect to the above, the motor control unit 107 may control the movement of the arm 3 such that the movement amount of the arm 3 associated with the flapping increases as the above-described shift increases. This is because the user 200 can be made to recognize that the above-described shift increases and that the correction of the orientation, position, and pose of the robot 100 by the user 200 is incorrect. For example, when the sum of the first shift amount and the second shift amount is greater than or equal to the sum of the previous first shift amount and the second shift amount indicated by the previous shift amount information 151 stored in the storage unit 103, the motor control unit 107 sets the movement amount of the arm 3 for flapping to be greater than or equal to the previous movement amount indicated by the previous movement amount information 152 stored in the storage unit 103. The increase amount of the movement amount may be predetermined and stored in the storage unit 103 or the like. Here, the motor control unit 107 may lengthen the flapping time as the shift of the vital sensor 14 relative to the user 200 increases.

As described above, the robot 100 can assist correction of the holding state of the robot 100 by the user 200 and reduce the shift of the vital sensor 14 relative to the user 200.

The motor control unit 107 may notify the user 200 that the vital sensor 14 is shifted relative to the user 200 by an operation other than the flapping of the arm 3. For example, the motor control unit 107 may control the operation of the robot 100 by flapping the leg 4 or moving the head 2 forward, backward, left, and right.

In addition to the control for notifying the user 200 that the vital sensor 14 is shifted relative to the user 200, the motor control unit 107 may perform control to change the orientation, position, and pose of the robot 100 itself. That is, the motor control unit 107 may change at least one of the orientation, position, or pose of the vital sensor 14 relative to the user 200 by changing at least one of the orientation, position, or pose of the robot 100. For example, the motor control unit 107 may control the operation of the robot 100 such that the robot 100 moves the arm 3 and the leg 4, the robot 100 itself crawls and moves on the body of the user 200, and the robot 100 itself changes its orientation and pose from the state of being held by the user 200. In this case, the robot 100 can also reduce the shift of the vital sensor 14 relative to the user 200.

The shift amount information acquisition unit 110 may output the shift amount information M1 to the display control unit 112. For example, when at least one of the orientation, position, or pose of the vital sensor 14 relative to the user 200 is shifted from a predetermined state, the display control unit 112 may output a command N2 to the display 24 through the output unit 109 to display an image expressing a negative emotion, such as "troubled eyes" or "angry eyes". The display control unit 112 can notify the user of the shift from the predetermined state by displaying the image representing a negative emotion on the display 24. After starting to display the image representing a negative emotion, the display control unit 112 stops the display after a predetermined time elapses. The user 200 recognizes that the holding state of the robot 100 is not appropriate by visually recognizing the image displayed on the display 24, and corrects at least one of the orientation, position, or pose of the robot 100. The orientation, position, and pose of the vital sensor 14 relative to the user 200 are corrected according to the correction of the orientation, position, and pose of the robot 100. When the orientation, position, and pose of the vital sensor 14 relative to the user 200 are in the predetermined state, the display control unit 112 may cause the display 24 to display an image representing a positive emotion, such as "smiling eyes" to notify the user that the predetermined state has been reached.

The shift amount information acquisition unit 110 may output the shift amount information M1 to the sound control unit 113. For example, when at least one of the orientation, position, or pose of the vital sensor 14 relative to the user 200 is out of the predetermined state, the sound control unit 113 may output, to the speaker 25, a command N3 to output a sound corresponding to the shift from the predetermined state. The sound control unit 113 can notify the user of the shift from the predetermined state by outputting a sound from the speaker 25. The sound control unit 113 starts outputting a sound corresponding to the shift from the predetermined state and stops outputting the sound after a predetermined time elapses. The user 200 recognizes that the holding state of the robot 100 is not appropriate by listening to the sound output from the speaker 25, and corrects at least one of the orientation, position, or pose of the robot 100. The orientation, position, and pose of the vital sensor 14 relative to the user 200 are corrected according to the correction of the orientation, position, and pose of the robot 100. When the orientation, position, and pose of the vital sensor 14 relative to the user 200 are in the predetermined state, the sound control unit 113 may output a sound corresponding to the predetermined state to the speaker 25 to notify the user of it. Additionally, the sound control unit 113 may assist correction of the holding state of the robot 100 by the user 200, by outputting a larger sound as the shift of the vital sensor 14 relative to the user 200 increases, or by outputting a smaller sound as the shift decreases.

The shift amount information acquisition unit 110 may output the shift amount information M1 to the light emission control unit 114. For example, when at least one of the orientation, position, or pose of the vital sensor 14 relative to the user 200 is out of a predetermined state, the light emission control unit 114 may output, to the right-cheek light 26a and the left-cheek light 26b, a command N4 to change the light emission states of the right-cheek light 26a and the left-cheek light 26b. The light emission control unit 114 can notify the user of the shift from the predetermined state by changing the light emission states of the right-cheek light 26a and the left-cheek light 26b. For example, the light emission control unit 114 can notify the user of the shift from the predetermined state by flashing the light emission of the light 26, making the light emission darker than the brightness when there is no shift from the predetermined state, or changing a color of the light emission with respect to the color when there is no shift from the predetermined state. After starting the light emission state change operation, the light emission control unit 114 stops the light emission state change operation after a predetermined time elapses. The user 200 recognizes that the holding state of the robot 100 is not appropriate by visually recognizing the light emission state of the light 26, and corrects at least one of the orientation, position, or pose of the robot 100. The orientation, position, and pose of the vital sensor 14 to the user 200 are corrected according to the correction of the orientation, position, and pose of the robot 100. When the orientation, position, and pose of the vital sensor 14 relative to the user 200 are in the predetermined state, the light emission control unit 114 may notify the user of the no shift from the predetermined state by the light emission state of the light 26. Additionally, the light emission control unit 114 may assist correction of the holding state of the robot 100 by the user 200, by darkening the light emission as the shift of the vital sensor 14 relative to the user 200 increases, or brightening the light emission as the shift decreases.

The robot 100 may notify the user 200 that at least one of the orientation, position, or pose of the vital sensor 14 is shifted by controlling at least one operation of the display 24, the light 26, the arm 3, or the speaker 25.

The shift amount information acquisition unit 110 may output the shift amount information M1 to the adjustable mechanism control unit 111. For example, when there is a shift of the vital sensor 14 relative to the user 200, the adjustable mechanism control unit 111 outputs, to the adjustable mechanism 18 via the output unit 109, a command N5 to control the operation of the adjustable mechanism 18. The command N5 corresponds to the mechanism control signal. By controlling the operation of the adjustable mechanism 18, the adjustable mechanism control unit 111 may change at least one of the orientation, position, or pose of the vital sensor 14 relative to the user 200. In this case, the robot 100 can also reduce the shift of the vital sensor 14 relative to the user 200.

In addition to the above, the vital sensor 14 may incorporate a mechanism for adjusting a transmission direction of the transmitted wave Ms or a reception direction of the reflected wave Mr in FIG. 5. The controller 13 may control an operation of the mechanism in the vital sensor 14 such that at least one of the orientation, position, or pose of the vital sensor 14 relative to the user 200 changes. The controller 13 can change a detection direction in which the vital sensor 14 detects the biological information by controlling the operation of the mechanism. Therefore, in the present specification, "the orientation, position, and pose of the vital sensor 14 changes" includes "the direction in which the vital sensor 14 detects the biological information changes". In the present specification, the shift amount information M1 includes information on the shift amount of the vital sensor 14 relative to the user 200 from a predetermined state in the detection direction.

### <Example of Process by the Controller 13>

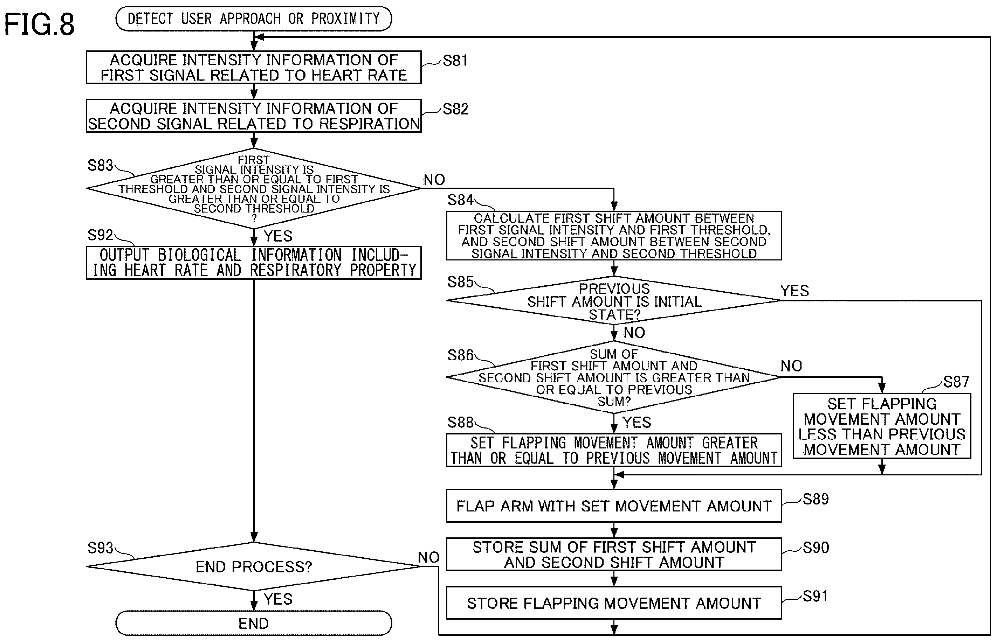

FIG. 8 is a flowchart illustrating a process by the controller 13. FIG. 8 illustrates an example of the process by the controller 13 to acquire the biological information from the user 200 by the vital sensor 14 in the robot 100. The controller 13 starts the process of FIG. 8 when the proximity detector 108 detects the contact or proximity of the user 200 with respect to the robot 100. Hereinafter, the description is provided with reference to the functional configuration of FIG. 7.

First, in step S81, the controller 13 acquires the intensity information of the first signal u1 related to the heart rate detected by the vital sensor 14 by the shift amount information acquisition unit 110.

Next, in step S82, the controller 13 acquires the intensity information of the second signal u2 related to the respiration detected by the vital sensor 14 by the shift amount information acquisition unit 110. Here, with respect to the processing of steps S81 and S82, the order may be suitably changed, or both may be performed in parallel.

Subsequently, in step S83, the controller 13 determines, by the shift amount information acquisition unit 110, whether the intensity of the first signal u1 is greater than or equal to the first threshold and the intensity of the second signal u2 is greater than or equal to the second threshold.

If it is determined in step S83 that the intensity of the first signal u1 is not greater than or equal to the first threshold and the intensity of the second signal u2 is not greater than or equal to the second threshold (step S83, NO), in step S84, the controller 13 calculates, by the shift amount information acquisition unit 110, the first shift amount between the intensity of the first signal u1 and the first threshold and the second shift amount between the intensity of the second signal u2 and the second threshold. The shift amount information acquisition unit 110 outputs the shift amount information M1 including the information on the first shift amount and the information on the second shift amount to the motor control unit 107.

Subsequently, in step S85, the controller 13 determines, by the motor control unit 107, whether the previous shift amount is in the initial state, that is, whether the sum of the first shift amount and the second shift amount is calculated for the first time in the process of FIG. 8.

If it is determined in step S85 that the previous shift amount is the initial state (step S85, YES), the controller 13 sets, by the motor control unit 107, the initial value of the predetermined movement amount as the movement amount of flapping. Then, the process transitions to step S89.

If it is determined in step S85 that the previous shift amount is not the initial state (step S85, NO), in step S86, the controller 13 determines, by the motor control unit 107, whether the sum of the first shift amount and the second shift amount is greater than or equal to the sum of the previous first shift amount and second shift amount indicated by the previous shift amount information 151 stored in the storage unit 103.

If it is determined in step S86 that the sum of the first shift amount and the second shift amount is not greater than or equal to the sum of the previous first shift amount and the second shift amount (step S86, NO), in step S87, the controller 13 sets, by the motor control unit 107, the movement amount of flapping less than the previous movement amount. Thereafter, the controller 13 transitions to step S89 of the process.

If it is determined in step S86 that the sum of the first shift amount and the second shift amount is greater than or equal to the previous sum of the first shift amount and second shift amount (step S86, YES), in step S88, the controller 13 sets, by the motor control unit 107, the movement amount of flapping greater than the previous movement amount.

Subsequently, in step S89, the controller 13 outputs, by the motor control unit 107 to the servo motor 35 via the output unit 109, the command N1 for "flapping" the arm 3. The motor control unit 107 notifies the user 200 that there is a shift by "flapping" the arm 3 via the servo motor 35. The motor control unit 107 stops the flapping after a predetermined time elapses since the start of the flapping operation of the arm 3.

Subsequently, in step S90, the controller 13 stores, by the storage unit 103, the sum of the first shift amount and the second shift amount as the previous shift amount information 151.

Subsequently, in step S91, the controller 13 stores, by the storage unit 103, the movement amount of flapping as the previous movement amount information 152. Thereafter, the controller 13 performs the processing after step S81 again. Here, with respect to the processing from step S89 to step S91, the order may be suitably changed, or they may be performed in parallel.

If it is determined in step S83 that the intensity of the first signal u1 is greater than or equal to the first threshold and that the intensity of the second signal u2 is greater than or equal to the second threshold (step S83, YES), in step S92, the controller 13 outputs, by the shift amount information acquisition unit 110 to the external device via the output unit 109, the biological information B including the heart rate and the respiratory characteristic.

Subsequently, in step S93, the controller 13 determines whether to end the process. For example, the controller 13 may determine to end the process if a predetermined time has elapsed, and may determine not to end the process if the predetermined time has not elapsed. Alternatively, the controller 13 may determine to end the process if it is detected that the user 200 is neither in contact with nor in proximity to the robot 100, and may determine not to end the process if it is detected that the user 200 is in contact with or in proximity to the robot 100. However, the method of determining whether to end the process may be as described above.

If, in step S93, it is determined not to end the process (step S93, NO), the controller 13 performs the processing after step S81 again. If it is determined to end the process in step S93 (step S93, YES), the controller 13 ends the process. When ending the process, the controller 13 may replace the previous shift amount information 151 and the previous movement amount information 152 with the initial values, or may update the initial values by using the previous shift amount information 151 and the previous movement amount information 152 at the time of the ending as the initial values.

As described above, the controller 13 can perform the process of acquiring the biological information from the user 200 by the vital sensor 14 in the robot 100. Here, the control of flapping the arm 3 is exemplified, but the flowchart of FIG. 8 can also be applied to the process of controlling the operation of the display 24, the speaker 25, the light 26, or the adjustable mechanism 18 by replacing the process by the motor control unit 107 with the process by the display control unit 112, the sound control unit 113, the light emission control unit 114, or the adjustable mechanism control unit 111.

### <Example of Holding State of Robot 100 by User 200>

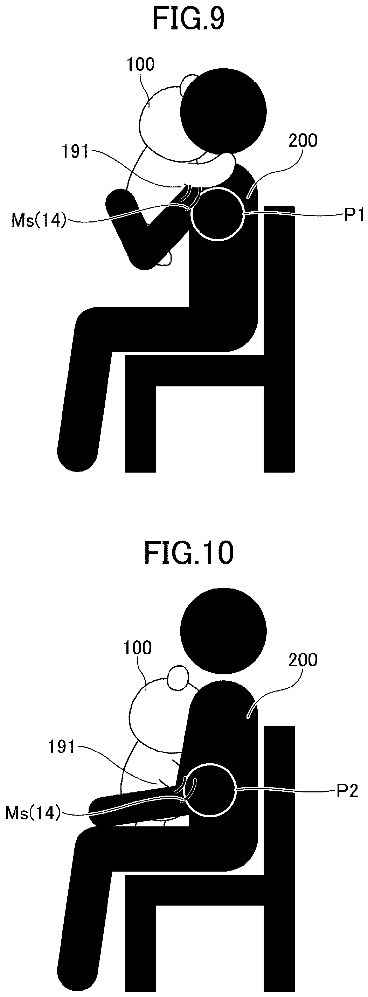

FIGS. 9 and 10 are diagrams illustrating the holding state of the robot 100. FIG. 9 illustrates a first example, and FIG. 10 illustrates a second example.

In FIG. 9, the user 200 holds the robot 100 such that an abdomen 191 of the robot 100 is positioned near the user's chest P1. The measurement position where the vital sensor 14 acquires the biological information from the user 200 is mainly near the user's chest P1 of the user 200. The transmitted wave Ms is mainly emitted toward the user's chest P1.

In FIG. 10, the user 200 holds the robot 100 such that the abdomen 191 of the robot 100 is positioned near the user's abdomen P2. The measurement position where the vital sensor 14 acquires the biological information from the user 200 is mainly near the user's abdomen P2 of the user 200. The transmitted wave Ms is mainly transmitted toward the user's abdomen P2.

The following Table 1 indicates the states of the first signal u1 related to the heart rate and the second signal u2 related to the respiration in the states of the first example illustrated in FIG. 9 and the second example illustrated in FIG. 10. "⊚" indicates that the signal state is the best, "○" indicates that the signal state is good, and "△" indicates that the signal state is normal. As indicated in Table 1, in the first example, the states of the first signal u1 related to the heart rate and the second signal u2 related to the respiration are both good. In the second example, the signal intensity of the second signal u2 related to the respiration is high and the signal state is the best because the biological information is mainly acquired from the user's abdomen P2 of the user 200. Conversely, the signal intensity of the first signal u1 related to the heart rate is low and the signal state is normal, and thus post-processing of the signal is required.

**[Table 1]**

| | FIRST EXAMPLE | SECOND EXAMPLE |
|---|---|---|
| MEASUREMENT LOCATION | CHEST | ABDOMEN |
| HEART RATE | ○ | Δ |
| RESPIRATION | ○ | ⊚ |

As described above, the biological information to be acquired changes depending on the measurement position, and thus it is preferable to devise the arrangement of the vital sensor 14 in the robot 100 in consideration of a use scene of the robot 100. For example, assuming that the user 200 holds the robot 100 on the knee, which is suitable for holding the robot 100, it is preferable to arrange the vital sensor 14 on the head of the robot 100 so that the biological information can be easily acquired from the user's chest P1 of the user 200. Alternatively, it is preferable to devise the arrangement of the vital sensor 14 suitable for two holding states of the inward-facing orientation and the forward-facing orientation.

### <Main Operation Effect of Robot 100>

As described above, the robot 100 includes the exterior member 10, the vital sensor 14 (the detector), and the controller 13. The controller 13 controls the operation of at least one of the robot 100 or the vital sensor 14 such that at least one of the orientation, position, or pose of the vital sensor 14 relative to the user 200 changes based on the biological information B acquired by the vital sensor 14. The biological information of the user is acquired in a non-contact manner using electromagnetic waves, and thus a sense of restraint or resistance felt by the user 200 is reduced, and the biological information can be naturally acquired. Additionally, in the present embodiment, at least one of the orientation, position, or pose of the vital sensor 14 relative to the user 200 is changed so that highly reliable biological information can be acquired when highly reliable biological information cannot be acquired because the orientation, position, or pose of the vital sensor 14 relative to the user 200 is not appropriate, or when highly reliable biological information cannot be acquired due to the presence of a shielding object, such as a cloth or baggage of the user 200. With this, in the present embodiment, the robot 100 that can naturally acquire highly reliable biological information can be provided. Here, the "reliability of biological information" can be determined, by setting a predetermined threshold value for a signal related to biological information, based on whether the signal related to the biological information exceeds the threshold value. In this case, the threshold value may be set for, for example, the peak intensity of the waveform of the signal or the S/N ratio.

Additionally, in the present embodiment, the controller 13 controls the operation of the robot 100 so as to notify the user 200 that at least one of the orientation, position, or pose of the vital sensor 14 relative to the user 200 is shifted from the predetermined state. When the user corrects the holding state of the robot 100 according to the operation of the robot 100, the robot 100 can naturally acquire highly reliable biological information. Here, in the present embodiment, the information on the heart rate and the respiration is mainly exemplified as the biological information, but the present embodiment can be applied to biological information other than heart rate and the respiration.

Although the preferred embodiments have been described in detail above, the present invention is not limited to the above-described embodiments, and various modifications and substitutions can be made to the above-described embodiments without departing from the scope of the appended claims.

Additionally, the numerals, such as ordinal numbers and quantities used in the description of the above-described embodiments are all examples for specifically describing the technique of the present invention, and the present invention is not limited to the numerals described as examples. Additionally, the connection relationship between the constituent elements is an example for specifically describing the technique of the present invention, and the connection relationship for realizing the functions of the present invention is not limited thereto.

The robot according to the present embodiment is particularly suitable for use in promoting oxytocin secretion and providing comfort (sense of security or self-affirmation) to a working adult living alone, a senior person whose child has moved out, a frail elderly person who is a target of home healthcare, or the like. However, the present invention is not limited to this use, and can be used to provide comfort to various users.

Aspects of the present disclosure are as follows, for example.
<1> A robot configured to acquire, in contact with or in proximity to a user, biological information of the user, the robot including:
   an exterior member;
   a detector configured to acquire the biological information by using an electromagnetic wave; and
   a controller configured to control, based on the biological information acquired by the detector, an operation of at least one of the robot or the detector, such that at least one of an orientation, position, or pose of the detector relative to the user changes.
<2> The robot as described in <1>, wherein the controller controls the operation of the robot so as to inform the user that the at least one of the orientation, position, or pose of the detector relative to the user is shifted from a predetermined state.
<3> The robot as described in <2>, wherein the controller controls the operation of the robot such that a movement amount of the robot decreases as the shift of the at least one of the orientation, position, or pose of the detector relative to the user from the predetermined state decreases.
<4> The robot as described in <2>, wherein the controller controls the operation of the robot such that a movement amount of the robot increases as the shift of the at least one of the orientation, position, or pose of the detector relative to the user from the predetermined state increases.
<5> The robot as described in any one of <1> to <4>, wherein the controller controls an operation of at least one of a display provided on an eye of the robot, a light emitter provided on a head of the robot, a drive section including an arm connected to a robot body in the robot to be relatively displaceable, or a sound generator provided in the robot.
<6> The robot as described in any one of <1> to <5>, wherein the biological information includes at least one of a pulse, a blood pressure, a heartbeat, or respiration.
<7> The robot as described in any one of <1> to <6>, wherein the controller changes at least one of the orientation, position, or pose of the detector relative to the user by changing at least one of an orientation, position, or pose of the robot.
<8> The robot as described in any one of <1> to <7>, including an adjustable mechanism configured to support the detector such that the at least one of the orientation, position, or pose of the detector is adjustable,
   wherein the controller controls an operation of the adjustable mechanism to change the at least one of the orientation, position, or pose of the detector relative to the user.

This application is based on and claims priority to Japanese Patent Application No. 2022-156761 filed in the Japan Patent Office on September 29, 2022, the entire contents of which are incorporated herein by reference.

### Description of reference symbols

1 trunk
2 head
2a right eye
2b left eye
2c mouth
2d right cheek
2e left cheek
3 arm
3a right arm
3b left arm
4 leg
4a right leg
4b left leg
5 nose
10 exterior member
11 camera (an example of a detector)
12 tactile sensor
13 controller
14 vital sensor (an example of a detector)
141 microwave transmitter
142 microwave receiver
15 battery
16 trunk frame
17 trunk mounting base
18 adjustable mechanism
181 linear motion mechanism
182 rotation mechanism
183 mechanism driving section
21 first capacitive sensor
22 head frame
23 head mounting base
24 display
24a right-eye display
24b left-eye display
25 speaker (an example of a sound generator)
26 light (an example of a light emitter)
26a right-cheek light
26b left-cheek light
27 head connection mechanism
31 second capacitive sensor
32a right arm frame
32b left arm frame
33 right arm mounting base
34a right arm connection mechanism
34b left arm connection mechanism
35 servo motor
35a right arm servo motor
35b left arm servo motor
35c head servo motor
35d right leg servo motor
35e left leg servo motor
41a right leg wheel
41b left leg wheel
42a right leg frame
42b left leg frame
44a right leg connection mechanism
44b left leg connection mechanism
100 robot
101 acquisition unit
102 communication control unit
103 storage unit
104 authentication unit
105 registration unit
106 start control unit
107 motor control unit
108 proximity detector
109 output unit
110 shift amount information acquisition unit
111 adjustable mechanism control unit
112 display control unit
113 sound control unit
114 light emission control unit
131 CPU
132 ROM
133 RAM
134 HDD/SSD
135 device connection I/F
136 communication I/F
150 registered information
151 previous shift amount information
152 previous movement amount information
191 abdomen
200 user
201 imaging light source
202 wavelength filter
203 lens
204 imaging element
A system bus
B biological information
C1 first capacitance signal
C2 second capacitance signal
F1a right shoulder frame
F2a right upper arm frame
F3a right elbow frame
F4a right forearm frame
F1b left shoulder frame
F2b left upper arm frame
F3b left elbow frame
F4b left forearm frame
F1c neck frame
F2c face frame
Im captured image
L irradiation light
Ms transmitted wave
Mr reflected wave
M1 shift amount information
M1a right shoulder servo motor
M2a right upper arm servo motor
M3a right elbow servo motor
M4a right forearm servo motor
M1b left shoulder servo motor
M2b left upper arm servo motor
M3b left elbow servo motor
M4b left forearm servo motor
M1c neck servo motor
M2c face servo motor
N1 to N5 command
P1 user's chest
P2 user's abdomen
R reflected light
S tactile signal
u1 first signal
u2 second signal

## Claims

1. A robot configured to acquire, in contact with or in proximity to a user, biological information of the user, the robot comprising:
an exterior member;
a detector configured to acquire the biological information by using an electromagnetic wave; and
a controller configured to control, based on the biological information acquired by the detector, an operation of at least one of the robot or the detector, such that at least one of an orientation, position, or pose of the detector relative to the user changes.

2. The robot as claimed in claim 1, wherein the controller controls the operation of the robot so as to inform the user that the at least one of the orientation, position, or pose of the detector relative to the user is shifted from a predetermined state.

3. The robot as claimed in claim 2, wherein the controller controls the operation of the robot such that a movement amount of the robot decreases as the shift of the at least one of the orientation, position, or pose of the detector relative to the user from the predetermined state decreases.

4. The robot as claimed in claim 2, wherein the controller controls the operation of the robot such that a movement amount of the robot increases as the shift of the at least one of the orientation, position, or pose of the detector relative to the user from the predetermined state increases.

5. The robot as claimed in claim 1 or 2, wherein the controller controls an operation of at least one of a display provided on an eye of the robot, a light emitter provided on a head of the robot, a drive section including an arm connected to a robot body in the robot to be relatively displaceable, or a sound generator provided in the robot.

6. The robot as claimed in claim 1 or 2, wherein the biological information includes at least one of a pulse, a blood pressure, a heartbeat, or respiration.

7. The robot as claimed in claim 1 or 2, wherein the controller changes at least one of the orientation, position, or pose of the detector relative to the user by changing at least one of an orientation, position, or pose of the robot.

8. The robot as claimed in claim 1 or 2, comprising an adjustable mechanism configured to support the detector such that the at least one of the orientation, position, or pose of the detector is adjustable,
wherein the controller controls an operation of the adjustable mechanism to change the at least one of the orientation, position, or pose of the detector relative to the user.

9. A robot configured to acquire, in contact with or in proximity to a user, biological information of the user, the robot comprising:
an exterior member;
a detector configured to acquire the biological information by using an electromagnetic wave; and
a controller configured to control an operation of at least one of the robot or the detector such that at least one of an orientation, position, or pose of the at least one of the robot or the detector relative to the user changes when reliability of the biological information acquired by the detector is less than a predetermined threshold.
